# EUROPEAN PATENT SPECIFICATION

(11) **EP 4 311 533 B1**
(45) Date of publication and mention of the grant of the patent: **10.09.2025**
(21) Application number: 23216121.6
(22) Date of filing: 30.11.2020
(51) Int. Cl.: A61M 1/38, A61J 1/10, A61M 1/36

(54) **A PRODUCTION DEVICE OF A MEDICATION COMPRISING PLATELETRICH PLASMA, AND A MANUAL PRODUCTION METHOD OF THE MEDICATION**
VORRICHTUNG ZUR HERSTELLUNG EINES MEDIKAMENTS MIT THROMBOZYTENREICHEM PLASMA UND MANUELLES HERSTELLUNGSVERFAHREN DES MEDIKAMENTS
DISPOSITIF DE PRODUCTION D'UN MÉDICAMENT COMPRENANT DU PLASMA RICHE EN PLAQUETTES ET PROCÉDÉ DE PRODUCTION MANUELLE DU MÉDICAMENT

(30) Priority: 04.12.2019 IT 201900022947
(43) Date of publication of application: 31.01.2024
(62) Divisional of application: 20830303.2
(73) Proprietor: Prometheus S.r.l., 43122 Parma (IT)
(72) Inventor: DELLA RAGIONE, Riccardo, Parma (IT); MICHELANGELI, Alice, Parma (IT); MENOZZI, Valentina, Altidona (FM) (IT)
(74) Representative: Dall'Olio, Christian

(56) References cited:
- EP-B1- 2 544 697
- US-A1- 2016 000 062

## Description

### FIELD OF THE INVENTION

The present invention relates to the technical sector of methods and means of production of a medication utilisable for the treatment of skin lesions and osteochondral or joint pathologies, and in particular for the regeneration of tissues.

### DESCRIPTION OF THE PRIOR ART

The medication is obtained by combining a blood derivative with suitable biomaterials that are bioresorbable. When of an autologous type, the medication is created only at point of need, following a collection of blood from the patient to be treated. The blood derivative contained therein is a platelet-rich plasma (thrombocytes) which is also called PRP, using the acronym. The PRP is obtained by centrifugation of blood and has a high concentration of growth factors. For this reason it has high effectiveness in the regeneration of tissues.

In the medical field, a connector called a "needle free connector" is known, which is interposable between a medical device for collection and/or storage and a manual collecting and injecting medical device of a liquid to place the two devices in fluid communication without altering the relative sterility thereof. The first of these can be a medical container for collecting or storing a biological liquid (blood, plasma, urine etc..) or a medical liquid (saline solution, pharmacological system etc.). The collection and injection device can comprise a cylinder-piston system and relative connection means, and typically a needle-free syringe. The needle free connector, at a first longitudinal end thereof, is engageable with an opening of the medical device for collection and/or storage and defines internally thereof a channel having a relative longitudinal axis and comprises, at a second longitudinal end, a compression valve. The compression valve is constituted by an elastically longitudinally-deformable element respective to the axis thereof. The connector is conformed so that, when the second longitudinal end is free, the valve obstructs the channel, preventing the passage of liquids and airborne bodies. The needle free connector is also conformed so that, when the second longitudinal end of the needle free connector engages with the connecting means of the manual collecting and injecting medical device, the valve is compressed, opening the channel and enabling a liquid to flow.

Typically, to realise the medications comprising PRP, the PRP is mixed with a gelling agent and optionally the gel obtained thus can be supported on a support layer made of a bio-resorbable and porous material also known as a "scaffold"; this simplifies the topical application of the medication.

US2016000062A1discloses a large multilayer flexible pouch made from bonding and sealing multiple layers of polymeric materials. A first layer of the polymeric material is bonded and sealed with second Layer on two edges to form inner compartment. The second layer and a third layer of the polymeric material is bonded and sealed at both edges to form an outer compartment which is bonded and sealed with a needle-free swabable connector at one end and is open at the other end. The inner and outer compartments can are connected. Alternatively, the inner and outer compartments are not connected. One or more sheets of tissue, membrane, or sponge with or without cells may be loaded into the inner compartment from the needle-free swabable connector. Another sheet of tissue, membrane, or sponge may be loaded into the outer compartment. After the tissue and/or cellular material are loaded, additional biological fluids, such as preservation solutions or biological solutions, may be added from either end of the pouch. Both the inner and outer pouch will be sealed through the opening at end. Sheet in the outer compartment and sheet in the inner compartment may be the same or different material.

From the above, in relation to the means and methods of production of the medication, the need to manually produce a medication comprising PRP strongly emerges in which the platelets are uniformly diffused, without any need to operate under a fume hood to maintain the sterility of the PRP and the medication with simple procedures and without expensive equipment.

Further, an especially known need is to minimise the possibility of contamination of the PRP produced and thus the medication obtained using the PRP, especially when a medication in gel form is obtained following the addition of calcium salts and incubation for at least 30 minutes in order to obtain the gelling of the PRP; since if this process took place in an environment that was not closed and sterile, the risk of contamination of the PRP with particles and micro-organisms would be high.

### SUMMARY OF THE INVENTION

The aim of the present invention consists in reducing and/or obviating the above-cited disadvantages with respect to the means and method of production of a medication comprising platelet-rich plasma.

In particular, the present invention has the aim of being able to produce a medication which comprises PRP, in which the platelets are re-suspended homogeneously, avoiding external contaminations without having to operate under a fume hood, using laboratory centrifuges of a known type and with modest costs. A further aim of the invention is to provide means and method for obtaining the medication produced with the PRP by using a closed and sterile hydraulic circuit with the aim of excluding any form of contamination due to the opening of a hydraulic circuit or a container.

These aims and objectives are attained by a production device of a medication comprising platelet-rich plasma, comprising platelet-rich plasma, and a manual production method of a medication, which are respective in accordance with claim 1 and claim 23, appended to the present description. The present invention enables being able to use a common bench centrifuge utilisable in the above-mentioned and known PRP production procedures that can be used in the production of a medication which comprises PRP. It is clear that, according to the invention, it is possible to produce the medication in a closed circuit and in conditions that prevent contamination thereof. Therefore, the presence of a fume hood is superfluous.

Although not expressly indicated, when reference is made to "a medical bag", it is to be understood that the medical bag is for the collection of biological liquids. This medical bag preferably has walls made of PVC.

### DESCRIPTION OF THE PREFERRED EMBODIMENTS

The characteristics of the invention will be described in the following in which some preferred but not exclusive embodiments of the production device of a medication comprising platelet-rich plasma and the manual production of the medication according to the invention and with reference to the appended table of drawings, in which:

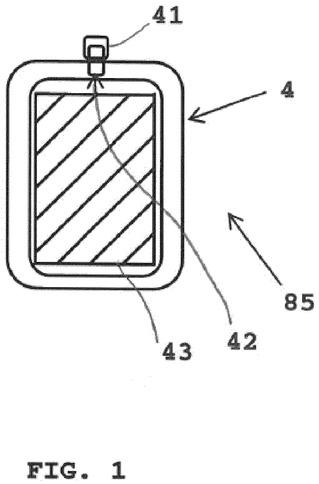

- figure 1 is a schematic front view of a production device of a medication comprising platelet-rich plasma (PRP) in accord to the invention. BRIEF

### DESCRIPTION OF THE DRAWINGS:

With reference to the figure, reference numeral (85) denotes a production device of a medication comprising platelet-rich plasma (PRP) according to the invention.

The production device (85) of a medication comprising platelet-rich plasma according to the invention, is sterile and comprises:
- a medical bag (4) for collection of biological liquids having a relative first wall and a relative second wall, opposite one another and defining a relative housing, which is internal and accessible from a opening (42) of the first medical bag (4),
- a needle free connector (41) arranged in and engaged with the opening (42), in such a way that a relative compression valve is arranged distally to the housing;
- a support element (43) (preferably planar) which comprises (or preferably is constituted by): a first support layer made of a bio-resorbable material, preferably porous material; a second layer, in a second bio-resorbable material, arranged on the first layer, wherein the second bio-resorbable material is a polymeric sponge or is a substance selected from a group constituted by: sodium alginate, gelatin, collagen and/or chitosan and combinations thereof and wherein the medical support is contained in the housing and is arranged with the first layer in contact with the first wall and with the second layer in contact with the second internal wall.

The first support layer thus constitutes a polymeric scaffold and can be advantageously obtainable by 3D printing. This enables controlling the relative thickness and dimension of the relative pores, if porous, which advantageously have dimensions comprised between 0.2 and 2 mm, preferably comprised between 0.5 and 1 mm.

In particular, when porous, the first layer degrades before a non-porous layer and guarantees air permeability, thus facilitating healing of wounds. The grid structure also represents an ideal support for the growth of cells during the regenerating process.

In a preferred embodiment, the first support layer has a thickness comprised between 5 and 500 µm, preferably comprised between 5 and 350 µm and more preferably 150 µm. It is worthy of note that a first layer having a porosity comprised between 0.2 and 2 mm, preferably comprised between 0.5 and 1 mm and a relative thickness of 150 µm, is able to support and/or contain a mixture of the second bio-resorbable material and the PRP which forms by actuating the manual production method of a medication set out in the following. The mixture separating from the first layer is thus avoided. The first bio-resorbable material, preferably porous material, can be: polycaprolactone (PLC), polylactic glycolic acid (PLGA), polyglycolic acid (PGA), and polyvinyl alcohol (PVA), polylactic acid (PLA), NYLON 680, polypropylene (PP), polyethylene (PE), polystyrene, high-impact polystyrene (HIPS), polycarbonate (PC), polyester ether ketone (PEEK), polyetherimide (PEI) and polysulphone (PSU) The preferred embodiments are those in which the first bio-resorbable material is polylactic acid (PLA).

The second layer can have a relative thickness comprised between 1 mm and 10 mm, preferably between 1 mm and 5 mm. The second layer is preferably constituted by the polymeric sponge, preferably made of a substance chosen from the group constituted by: sodium alginate, gelatin, collagen and/or chitosan and combinations thereof. More preferably, this substance is gelatin and is advantageously pork skin gelatin to facilitate absorption of the PRP and the gradual release of growth factors following the application of the medication on the wound. The polymeric sponge advantageously has a relative degree of porosity of 40-60 %, preferably of 45 - 55 %. This is because, in this case, the polymeric sponge absorbs and distributes the PRP more uniformly, enabling the obtaining of a medication with a greater uniformity of distribution of the PRP and starting from a collection of a volume of blood from the patient of a small entity, while maintaining a high concentration of platelets in the final medication.

A manual production method of a medication comprising platelet-rich plasma, according to the invention, comprises following steps:
a) predisposing platelet-rich plasma;
b) manually injecting the platelet-rich plasma, optionally added-to by a coagulating agent, into the housing of a production device (85) of a medication, according to the invention:
c) homogeneously distributing the platelet-rich plasma in the support element (43) of the production device (85) of a medication, preferably by compression of the support element (43), and of the platelet-rich plasma injected, contained in the housing between the first wall and the second wall.

Step c), relating to distribution, enables obtaining a medication in which the PRP is also homogeneously distributed on large surfaces with small volumes of PRP, to cover large lesions.

**It** is further of considerable importance that the manual production method of the medication enables directly obtaining a package (not illustrated) comprising the medication enabling the avoiding of further packaging steps when it is not expected to have to use the medication produced immediately. The package comprises a packaging and a medication containing in the packaging, where the medication comprises platelet-rich plasma, a support element (43) which in time comprises: a first support layer made of a first bio-resorbable and porous material; a second layer, in a second bio-resorbable material, arranged on the first layer, wherein the second bio-resorbable material is a polymeric sponge or is a substance selected from a group constituted by: sodium alginate, gelatin, collagen and/or chitosan and combinations thereof, wherein the platelet-rich plasma is dispersed in the second bio-resorbable material, and wherein the second layer is arranged on the first layer. The package is characterised in that the packaging is a medical bag, preferably made of PVC, for collection of biological liquids having a relative first wall and a relative second wall, opposite one another and defining a relative internal housing; in that the first layer is in contact with the first wall; in that the second layer is in contact with the second internal wall, and in that the housing is accessible from an opening of the medical bag in which a needle free connector is arranged, in such a way that a relative compression valve is arranged distally to the housing of the medical bag. The preferred embodiments described in the foregoing in relation to the production device (85) and to the support element (43), to the first and second layer, to the first and a second bio-resorbable material are applied also to the package.

Purely by way of example some dimensions of some elements described herein are given. The medical bags for collection of biological liquids can have a relative housing of 70 ml.

## Claims

1. A production device (85) of a medication comprising platelet-rich plasma, the device being sterile and comprising:
- a medical bag (4) for collection of biological liquids having a relative first wall and a relative second wall, opposite one another and defining a relative housing, which is internal and accessible from a opening (42) of the first medical bag (4),
- a needle free connector (41) arranged in and engaged with the opening (42), in such a way that a relative compression valve is arranged distally to the housing;
a support element (43) which comprises: a first support layer made of a bio-resorbable material; a second layer, made of a second bio-resorbable material, wherein the second bio-resorbable material is a polymeric sponge or is a substance selected from a group constituted by: sodium alginate, gelatin, collagen and/or chitosan and combinations thereof, the production device (85) being **characterized in that** the second bio-resorbable material is arranged on the first layer, and **in that** the support element (43) is contained in the housing and is arranged with the first layer in contact with the first wall and with the second layer in contact with the second wall.

2. A production device (85) according to claim 1, wherein the first support layer made of a porous bio-resorbable material.

3. A manual *ex vivo* production method of a medication, which comprises platelet-rich plasma, the method comprising following step:
a) predisposing platelet-rich plasma from blood;
the manual production method being **characterized in that** it further comprises following steps:
b) manually injecting the platelet-rich plasma into the third housing of a production device (85) of a medication, according to claim 1-2;
c) homogeneously distributing the platelet-rich plasma in the support element (43) of the production device (85) of a medication, and of the platelet-rich plasma injected, contained in the housing between the first wall and the second wall.

4. A manual production method of a medication according to claim 3, which comprises platelet-rich plasma, wherein the step b) of manually injecting is as follows:
b) manually injecting the platelet-rich plasma added-to by a coagulating agent, into the housing of a production device (85) of a medication, according to claim 1.

5. A manual production method of a medication according to claim 3, which comprises platelet-rich plasma, wherein the step a) of homogeneously distributing is as follows:
c) homogeneously distributing the platelet-rich plasma in the support element (43) of the production device (85) of a medication, by compression of the support element (43), and of the platelet-rich plasma injected, contained in the housing between the first wall and the second wall,

## Patentansprüche

1. Vorrichtung (85) zur Herstellung eines Medikaments, das plättchenreiches Plasma enthält, wobei die Vorrichtung steril ist und umfasst:
- einen medizinischen Beutel (4) zum Sammeln von biologischen Flüssigkeiten mit einer relativen ersten Wand und einer relativen zweiten Wand, die einander gegenüberliegen und ein relatives Gehäuse bilden, das innen liegt und von einer Öffnung (42) des ersten medizinischen Beutels (4) aus zugänglich ist,
- einen nadelfreien Verbinder (41), der in der Öffnung (42) angeordnet und mit dieser in Eingriff steht, so dass ein relatives Kompressionsventil distal zum Gehäuse angeordnet ist;
ein Stützelement (43), das umfasst: eine erste Stützschicht aus einem bioresorbierbaren Material; eine zweite Schicht aus einem zweiten bioresorbierbaren Material, wobei das zweite bioresorbierbare Material ein polymerer Schwamm ist oder eine Substanz ist, die aus einer Gruppe ausgewählt ist, die besteht aus: Natriumalginat, Gelatine, Kollagen und/oder Chitosan und Kombinationen davon, wobei die Herstellungsvorrichtung (85) **dadurch gekennzeichnet ist, dass** das zweite bioabsorbierbare Material auf der ersten Schicht angeordnet ist und dass das Stützelement (43) in dem Gehäuse enthalten ist und mit der ersten Schicht in Kontakt mit der ersten Wand und mit der zweiten Schicht in Kontakt mit der zweiten Wand angeordnet ist.

2. Herstellungsvorrichtung (85) nach Anspruch 1, wobei die erste Trägerschicht aus einem porösen bioresorbierbaren Material besteht.

3. Manuelles Ex-vivo-Herstellungsverfahren für ein Medikament, das plättchenreiches Plasma umfasst, wobei das Verfahren folgenden Schritt umfasst:
a) Bereitstellen von plättchenreichem Plasma aus Blut;
wobei das manuelle Herstellungsverfahren **dadurch gekennzeichnet ist, dass** es ferner folgende Schritte umfasst:
b) manuelles Injizieren des plättchenreichen Plasmas in das dritte Gehäuse einer Vorrichtung (85) zur Herstellung eines Medikaments gemäß Anspruch 1-2;
c) homogenes Verteilen des plättchenreichen Plasmas in dem Trägerelement (43) der Vorrichtung (85) zur Herstellung eines Medikaments und des injizierten plättchenreichen Plasmas, das in dem Gehäuse zwischen der ersten Wand und der zweiten Wand enthalten ist.

4. Manuelles Herstellungsverfahren für ein Medikament gemäß Anspruch 3, das plättchenreiches Plasma umfasst, wobei der Schritt b) des manuellen Injizierens wie folgt ist:
b) manuelles Injizieren des mit einem Gerinnungsmittel versetzten plättchenreichen Plasmas in das Gehäuse einer Vorrichtung (85) zur Herstellung eines Medikaments gemäß Anspruch 1.

5. Manuelles Herstellungsverfahren für ein Medikament nach Anspruch 3, das plättchenreiches Plasma umfasst, wobei der Schritt a) des homogenen Verteilens wie folgt erfolgt:
c) das plättchenreiche Plasma in dem Trägerelement (43) der Vorrichtung (85) zur Herstellung eines Medikaments durch Zusammendrücken des Trägerelements (43) und des injizierten, in dem Gehäuse zwischen der ersten Wand und der zweiten Wand enthaltenen plättchenreichen Plasmas homogen verteilt wird.

## Revendications

1. Dispositif de production (85) d'un médicament comprenant du plasma riche en plaquettes, le dispositif étant stérile et comprenant :
- un sac médical (4) destiné à la collecte de liquides biologiques, comportant une première paroi relative et une deuxième paroi relative, opposées l'une à l'autre et définissant un logement relatif, qui est interne et accessible depuis une ouverture (42) du premier sac médical (4),
- un connecteur sans aiguille (41) disposé dans l'ouverture (42) et engagé avec celle-ci, de telle sorte qu'une valve de compression relative est disposée de manière distale par rapport au logement ;
un élément de support (43) qui comprend : une première couche de support constituée d'un matériau biorésorbable ; une deuxième couche, constituée d'un deuxième matériau biorésorbable, dans lequel le deuxième matériau biorésorbable est une éponge polymère ou est une substance choisie dans un groupe constitué par : l'alginate de sodium, la gélatine, le collagène et/ou le chitosane et des combinaisons de ceux-ci, le dispositif de production (85) étant **caractérisé en ce que** le deuxième matériau biorésorbable est disposé sur la première couche, et **en ce que** l'élément de support (43) est contenu dans le boîtier et est disposé avec la première couche en contact avec la première paroi et avec la deuxième couche en contact avec la deuxième paroi.

2. Dispositif de production (85) selon la revendication 1, dans lequel la première couche de support est constituée d'un matériau biorésorbable poreux.

3. Procédé de production manuelle ex vivo d'un médicament, qui comprend du plasma riche en plaquettes, le procédé comprenant l'étape suivante :
a) prédisposer du plasma riche en plaquettes à partir de sang ;
le procédé de production manuelle étant **caractérisé en ce qu'**il comprend en outre les étapes suivantes :
b) l'injection manuelle du plasma riche en plaquettes dans le troisième logement d'un dispositif de production (85) d'un médicament, selon les revendications 1-2 ;
c) la distribution homogène du plasma riche en plaquettes dans l'élément de support (43) du dispositif de production (85) d'un médicament, et du plasma riche en plaquettes injecté, contenu dans le logement entre la première paroi et la deuxième paroi.

4. Procédé de fabrication manuelle d'un médicament selon la revendication 3, qui comprend du plasma riche en plaquettes, dans lequel l'étape b) d'injection manuelle est la suivante :
b) injecter manuellement le plasma riche en plaquettes additionné d'un agent coagulant dans le logement d'un dispositif de fabrication (85) d'un médicament, selon la revendication 1.

5. Procédé de fabrication manuelle d'un médicament selon la revendication 3, qui comprend du plasma riche en plaquettes, dans lequel l'étape a) de distribution homogène est la suivante :
c) distribuer de manière homogène le plasma riche en plaquettes dans l'élément de support (43) du dispositif de production (85) d'un médicament, par compression de l'élément de support (43) et du plasma riche en plaquettes injecté, contenu dans le boîtier entre la première paroi et la deuxième paroi.
